# EUROPÄISCHE PATENTSCHRIFT

(11) **EP 4 360 578 B1**
(45) Veröffentlichungstag und Bekanntmachung des Hinweises auf die Patenterteilung: **20.08.2025**
(21) Anmeldenummer: 23204372.9
(22) Anmeldetag: 18.10.2023
(51) Int. Cl.: A61B 34/00, A61B 90/00

(54) **ENDOSKOPISCHES SYSTEM UND VERFAHREN**
ENDOSCOPIC SYSTEM AND METHOD
SYSTÈME ENDOSCOPIQUE ET PROCÉDÉ

(30) Priorität: 25.10.2022 DE 102022128268
(43) Veröffentlichungstag der Anmeldung: 01.05.2024
(73) Patentinhaber: Karl Storz SE & Co. KG, 78532 Tuttlingen (DE)
(72) Erfinder: Wagner, Sebastian, 78532 Tuttlingen (DE); Wenzler, Sebastian, 78532 Tuttlingen (DE)

(56) Entgegenhaltungen:
- US-A1- 2005 021 078
- US-A1- 2019 125 337
- US-A1- 2021 196 365

## Beschreibung

Ein Chirurg ist primär auf den Einsatz seiner Hände und Finger angewiesen, mit denen er bei offenen chirurgischen Eingriffen das Gewebe abtastet, spürt, palpiert etc. und viele Arbeitsschritte und Entscheidungen rein auf Basis seines taktilen Gefühls durchführt.

Aus der US 2021/0196365 A1 ist ein chirurgisches System mit einem chirurgischen Instrument, einem Generator, der dazu ausgelegt ist, einen Endeffektor mit Energie zu versorgen, und einem Prozessor, der dazu ausgelegt ist, ein Steuerprogramm auszuführen, um das chirurgische System zu betreiben, bekannt. Das chirurgische Instrument weist den Endeffektor mit einem ersten Maulteil und einem zweiten Maulteil auf. Mindestens eines der beiden Maulteile bewegt sich relativ zueinander zwischen einer offenen Position und einer geschlossenen Position. Das Gewebe wird zwischen dem ersten und dem zweiten Maulteil positioniert. Der Prozessor ist so konfiguriert, dass er einen ersten Parameter des chirurgischen Systems erfasst, mindestens eine Benutzereingabe erfasst und das Steuerprogramm in Reaktion auf den erfassten ersten Parameter und die mindestens eine Benutzereingabe modifiziert.

Die US 2005/0021078 A1 betrifft ein chirurgisches Instrument, insbesondere für die minimal invasive Chirurgie. Das Instrument weist Mittel auf, um eine auf das Arbeitselement des Instruments ausgeübte Kraft auf das Bedienelement zurückzuführen. Diese Mittel weisen mindestens einen ersten Kraftsensor zum Messen der auf das Arbeitselement ausgeübten Kraft, eine Steuereinheit und einen ersten Aktuator auf. Auf der Grundlage eines Signals des ersten Kraftsensors steuert die Steuereinheit zumindest den ersten Aktuator, um das Bedienelement zu steuern. Außerdem weisen die Mittel vorzugsweise einen ersten Positionssensor zum Messen der Position des Arbeitselements relativ zum Rahmen auf. Die Steuereinheit bestimmt vorteilhafterweise einen Widerstand, dem das Arbeitselement aufgrund des Vorhandenseins von Gewebe oder dergleichen ausgesetzt ist, auf dessen Grundlage die Steuereinheit zumindest den ersten Aktuator steuert.

Die US 2019/0125337 A1 betrifft ein modulares chirurgisches Instrument. Das modulare chirurgische Instrument weist eine Steuerungsschnittstelle, einen sich von der Steuerungsschnittstelle erstreckenden Schaft, einen sich von dem Schaft erstreckenden Endeffektor und eine Steuerschaltung auf. Die Steuerschaltung ist so konfiguriert, dass sie das an das modulare chirurgische Instrument angelegte elektrische Potential erfasst, bestimmt, ob das erfasste elektrische Potential über einem vorbestimmten Schwellenwert liegt, und den Betrieb des modularen chirurgischen Instruments anpasst, wenn das erfasste elektrische Potential den vorbestimmten Schwellenwert überschreitet.

Bei klassischen manuellen endoskopischen Eingriffen im Rahmen der minimal-invasiven Chirurgie (MIC) geht diese Art von Tastsinn nahezu ganz verloren. Der Chirurg spürt zwar einen Widerstand, aber die Möglichkeit all der feinen Unterscheidungen zwischen unterschiedlichen Gewebearten und Strukturen, die er mit seinen Händen intuitiv treffen könnte, gehen weitgehend bis komplett verloren. Aber der Chirurg kann zumindest auf Basis einer optischen Wahrnehmung über ein Bild auf einem Bildschirm noch Rückschlüsse ziehen. Hierzu zählt insbesondere eine im Bild erkennbare Greifwirkung, so dass der Chirurg die Greifkraft, mit der er das Gewebe greift, variieren kann.

Es wurden bisher schon verschiedene Konzepte beschrieben, wie die Gegenkraft des Gewebes (distal) direkt gemessen werden kann, z. B. über Kraftsensoren im Aktuator, insbesondere im Maulteil, Kraftsensoren in den Übertragungselementen, insbesondere in einer Zug- / DruckStange, oder Kraftsensoren an den Antriebselementen, wie dem Aktuatormotor. Es besteht auch die Möglichkeit, die Aktuatorkraft aus dem Ansteuerungsstrom für den Aktuatormotor abzuleiten.

Ebenso gibt es Ansätze, wie diese Information über diese Gegenkraft dem Benutzer (proximal) vermittelt werden kann, z. B. über das Erzeugen von einer Gegen- / Widerstandskraft in der Eingabe, aktiv über Antriebe, z.B. elektromotorisch, hydraulisch, elektromagnetisch, passiv über dämpfende bzw. bremsende Mechanismen oder über andere optische, haptische und / oder akustische Informationskanäle wie z.B. Bildgebung, Tongeber, Vibration, etc.

Die Idee ist, dass der Benutzer mit dieser Zusatzinformation (Gegenkraft) über feindosierte Greifbewegungen, das Gewebe abtasten kann, und ihm so quasi seine manuellen taktilen Fähigkeiten zurückgegeben werden
Ein echtes 1: 1 direktes Feedback umzusetzen, ist sowohl auf der (distalen) Instrumentenseite bzgl. der technischen Realisierung einer exakten, präzisen, reproduzierbaren, kalibrierten Kraftmessung (miniaturisiert wegen begrenzten Einbauräumen) als auch (proximal) an der Bedieneinheit bzgl. der technischen Umsetzung bzw. Erzeugung der gemessenen distalen Gegenkräfte technisch betrachtet sehr anspruchsvoll und führt zumindest derzeit noch zu unverhältnismäßig aufwändigen und komplexen technischen Lösungen, die dann, wenn überhaupt, nur ansatzweise dem Benutzer einen sensorische Kraftrückkopplung und einen Tastsinn vermitteln.

Insbesondere an der Betätigungseinheit zur Steuerung der Instrumentenbewegungen sind technische Lösungen, um ein realistisches "Fingertastgefühl" zu vermitteln, das dem tatsächlichen Greifen / Tasten mit den Fingern entsprechen würde, nach heutigem Stand der Technik / Wissensstand noch nicht wirklich in Sicht.

Es ist daher eine Aufgabe der vorliegenden Erfindung ein verbessertes endoskopisches System und ein verbessertes Verfahren zum Ansteuern eines Aktuators eines endoskopischen Systems, die dem Benutzer, trotz der bekannten Beschränkungen beim Erzielen eines realistischen Fingertastgefühls, ein intuitives Arbeiten mit der Hand und den Fingern ermöglicht. Dabei soll insbesondere die technische Realisierung nur eine vergleichsweise geringe Komplexität aufweisen.

Die Aufgabe wird gelöst durch ein endoskopisches System aufweisend einen Aktuator an einem distalen Ende des Systems, eine handbedienbare Betätigungseinheit an einem proximalen Ende des Systems zum Betätigen des Aktuators und Wählen einer Aktuatorkraft, mit der der Aktuator auf ein Objekt einwirken soll, wobei die Betätigungseinheit zwischen einer Anfangsstellung und einer Endstellung verlagerbar ist, einen Aktuatormotor, der dazu ausgebildet ist, den Aktuator zum Erreichen einer gewünschten Stellung anzusteuern, und eine Steuereinheit, die dafür ausgebildet ist, den Aktuatormotor: a) während einer Betätigung der Betätigungseinheit zwischen der Anfangsstellung und der Endstellung in Abhängigkeit von einer aktuellen Stellung der Betätigungseinheit zu betätigen, wenn eine vom Aktuator ausgeübte Aktuatorkraft einen Grenzwert nicht überschreitet, b) während einer Betätigung der Betätigungseinheit zwischen der Anfangsstellung und der Endstellung nicht weiter zu betätigen, wenn eine vom Aktuator ausgeübte Aktuatorkraft den Grenzwert erreicht oder überschreitet, und c) während einer Betätigung der Betätigungseinheit in der Endstellung in Abhängigkeit von einer auf die Betätigungseinheit ausgeübten Bedienkraft über den Grenzwert hinaus anzusteuern.

Bei einer Betätigung der Betätigungseinheit von der Anfangsstellung oder Nullstellung in Richtung der Endstellung wird der Aktuator entsprechend der aktuellen Stellung der Betätigungseinheit betätigt. Die vom Benutzer auf die Betätigungseinheit ausgeübte Bedienkraft ist dabei nicht maßgeblich. Insbesondere ist eine Gegenkraft, die der Benutzer in seiner Hand fühlt, nur gering oder sogar nicht spürbar. Die Betätigung des Aktuators hängt hier von der aktuellen Stellung ab, nicht von einer durch den Benutzer ausgeübten Bedienkraft. Die Betätigung der Betätigungseinheit zwischen der Anfangsstellung und der Endstellung findet insbesondere mit einer minimalen Bedienkraft statt.

Diese Betätigung wird aber nicht fortgesetzt, wenn eine vom Aktuator ausgeübte Aktuatorkraft den Grenzwert erreicht oder überschreitet. Das heißt, wenn der Grenzwert erreicht oder überschritten ist, führt eine fortschreitende Verlagerung der Betätigungseinheit nicht zu einer weiteren Betätigung des Aktuators. Vielmehr behält der Aktuator die aktuelle Position, und damit üblicherweise auch die von ihm ausgeübte Aktuatorkraft bei. Diese Art der Ansteuerung erfolgt, sofern die Endstellung noch nicht erreicht ist.

Wenn die Endstellung erreicht ist, ändert sich die Art der Ansteuerung. Ein fortgesetzter oder erhöhter Krafteinsatz an der Betätigungseinheit führt nun nicht mehr zu einer weiteren Verlagerung der Betätigungseinheit entlang des Wegs zwischen Anfangsstellung und Endstellung. Vielmehr wird nun die Bedienkraft erfasst, mit der die Betätigungseinheit in der Endstellung betätigt wird. In Abhängigkeit von der erfassten Bedienkraft wird der Aktuator weiter betätigt, so dass die von ihm ausgeübte Aktuatorkraft den Grenzwert überschreitet.

Eine bevorzugte Realisierung ist dabei wie folgt. In Abhängigkeit von der Bedienkraft wird ein neuer Maximalwert bestimmt, der den genannten Grenzwert überschreitet. Der Aktuator wird dann bis zu diesem neuen Maximalwert angesteuert. Mit einer weiteren Veränderung der Bedienkraft wird der Maximalwert wieder angepasst und der Aktuator wieder bis zu diesem neuen Maximalwert angesteuert. Der genannte Grenzwert, auch initialer Grenzwert genannt, wird bevorzugt als der Wert gewählt, bei dem das schwächste Gewebe nur minimal elastisch deformiert wird, nicht darüber hinaus. Der Maximalwert, der aus der Bedienkraft abgeleitet wird, übersteigt dann diesen Grenzwert.

Der Aktuator weist bei bevorzugten Ausgestaltungen eine servomotorische Ansteuerung auf. Die handbedienbare Betätigungseinheit kann sich bevorzugt an einem proximalen Ende des Systems befinden, wenn das System als handgeführtes Instrument ausgebildet ist. Die Betätigungseinheit kann aber auch losgelöst von dem eigentlichen Instrument beliebig positioniert sein kann, z.B. an einer Steuerkonsole.

Der Begriff proximal soll im Rahmen dieser Beschreibung so verstanden werden, dass es sich um eine Seite oder ein Ende des Systems handelt, das dem Benutzer nahe ist und an dem der Benutzer die Bedienung steuert. Wenn das endoskopische System als endoskopisches Instrument ausgeführt ist, kann das proximale Ende des Systems insbesondere in direkter mechanischer Verbindung mit dem distalen Ende des Systems stehen. Dabei kann es sich insbesondere um ein handgeführtes Instrument handeln, bei dem auch das distale Ende direkt durch die Handführung am proximalen Ende positioniert wird. Das endoskopische System kann aber auch als Robotersystem oder Telemanipulator ausgestaltet sein.

Die Erfinder haben im Rahmen der Erfindung erkannt, dass ein versierter Chirurg mit einiger Übung bei chirurgischen Eingriffen mit den manuellen Standard-Systemen die fehlende sensorische Rückkopplung soweit kompensieren kann, dass diese Eingriffe erfolgreich umgesetzt werden können und dass sich immer mehr endoskopische Eingriffe neben den offenchirurgischen Eingriffen etabliert und durchgesetzt haben.

Als einen Aspekt der Lösung haben die Erfinder identifiziert, dass der Chirurg mit diesen manuellen Standard-Systemen zwar kein sensorisches Feedback hat, aber er die applizierte Aktuatorkraft auf das Gewebe variieren und anhand der visuell beobachteten Wirkung auf das Gewebe dosieren kann. Das heißt, der Chirurg kann zwar über die Systemmechanik kaum fühlen, aber er kann über die Bedienkraft bzw. Handkraft die Kräfte kontrollieren und über die visuelle Beobachtung der Wirkung entsprechend adaptieren.

Diese Funktionalität der Kraftkontrolle ist bei einer servomotorischer Wegübertragung der Fingerbewegung auf den Aktuator, insbesondere auf Maulteilbewegungen, nicht mehr gegeben, was die Handhabung der Systeme mit servomotorischer Wegübertragung gegenüber manuell betätigten Standard-MIC-Systemen deutlich erschwert.

Somit bietet die Erfindung insbesondere eine Möglichkeit dafür, dem endoskopisch operierenden Chirurgen für servomotorisch angesteuerte Systembewegungen die gleiche Funktionalität zu bieten, wie er sie für manuell betätigte Systeme hat. Dabei soll der Chirurg insbesondere die Möglichkeit haben, die wirkende Aktuatorkraft zu variieren und anhand der visuell beobachteten Wirkung die Kräfte in Echtzeit anzupassen.

Die technische Lösung hierfür soll dabei gezielt nur eine Kraftvariation und Kontrolle der Kraftdosierung ermöglichen, und ganz bewusst das Ziel eines taktilen kraftsensorischen Feedbacks ausklammern. Dies vereinfacht die technische Realisierung, führt überraschenderweise aber dennoch zu einer vorteilhaften Bedienbarkeit.

Es wird so eine einfache und robuste technische Lösung ermöglicht, die ganz bewusst die komplexen aufwendigen technischen Lösungen für ein möglichst echtes sensorisches Feedback vermeidet. Die Erfinder haben erkannt, dass diese heute bekannten Lösungen das Versprechen für ein echtes taktiles Gefühl nicht einlösen können, gleichzeitig, aber aufwendig sind und ein hohes Risiko aufweisen, Falschinformationen zu vermitteln, die zu Fehlinterpretationen und Handlungen zu führen.

Mit anderen Worten, zwar wäre ein 1: 1 übertragenes taktiles Spüren des Gewebes der allgemeine Wunschtraum, aber hier soll für servomotorisch angetriebene Systeme zunächst einmal die Kraftkontrolle ermöglicht werden, wie sie rein manuell mechanisch angetriebene Systeme aufweisen. Die Erfinder haben erkannt, dass es für motorisch angetriebene Systembewegungen in einem ersten Schritt für den Benutzer bereits eine enorme Hilfe ist, wenn der Benutzer wie bei den konventionellen, rein manuell mechanisch angetriebenen Systemen die Kraftwirkung kontrollieren kann.

Ein Aspekt der Erfindung besteht darin, dass bei Systemen, bei denen Handbewegungen an der Betätigungseinheit über Servomotoren in Systembewegungen übertragen werden, insbesondere auf eine Kraftmessung der Gegenkraft des Gewebes an der distalen Seite des endoskopischen Systems ganz verzichtet wird.

Dafür sind insbesondere in der handbedienbaren Betätigungseinheit zusätzlich ein oder mehrere Kraftsensoren integriert sind, die die Kräfte messen, die der Benutzer auf die Betätigungseinheit zum Steuern des Systems ausübt. Über die Messung der Veränderung dieser auf die Betätigungseinheit vom Benutzer ausgeübten Kräfte, werden in vergleichbarer Weise die Bewegungskräfte der motorisierten Systembewegungen verändert.

Obwohl dieser Aufbau vergleichsweise einfach ist, ermöglicht er es auf überraschend vorteilhafterweise dem Benutzer auch bei motorisierten Systemen die Kräfte wie bei einem konventionellen manuellen MIC-System zu variieren und über die visuelle Beobachtung und Kontrolle der Wirkung auf das Gewebe anzupassen, z.B. beim Greifen von Gewebe. Insbesondere kann der Benutzer eine Greifkraft auf das Gewebe über eine manuell aufgebrachte Bedienkraft, insbesondere Fingerkräfte, dosieren und deren Wirkweise visuell überprüfen, insbesondere in Echtzeit. Ob die ausgeübte Bedienkraft an der Betätigungseinheit bzw. Eingabeeinheit tatsächlich genau der Aktuatorkraft, insbesondere Maulteil-Greifkraft, entspricht, ist nach Erkenntnis der Erfinder nicht maßgeblich. Entscheidend ist, dass sich die Aktuatorkraft bzw. Greifkraft über die vom Benutzer aufgebrachte Bedienkraft oder Schließkraft verändern lässt.

Dadurch wird die Aufgabe vollständig gelöst.

Bei einer vorteilhaften Ausgestaltung ist das System für eine Zangenfunktion ausgebildet, weist der Aktuator zwei Maulteile auf und ist das Betätigen des Aktuators ein Öffnen und Schließen der Maulteile.

Es wird davon ausgegangen, dass die Realisierung der Erfindung in diesem technischen Kontext besonders vorteilhaft ist, insbesondere bei einer Greifzange. Grundsätzlich kommen aber auch andere Systemarten in Frage, zum Beispiel solche, die eine Schere, eine variable Schlinge oder ein drehbares Element als Aktuator haben.

Bei einer weiteren vorteilhaften Ausgestaltung weist die Betätigungseinheit einen Kraftsensor auf, der dafür ausgebildet ist, zumindest in der Endstellung die auf die Betätigungseinheit ausgeübte Bedienkraft zu messen.

Auf diese Weise lässt sich die ausgeübte Bedienkraft verhältnismäßig einfach messen.

Bei einer weiteren vorteilhaften Ausgestaltung weist der Kraftsensor ein Biegeelement zur Messung einer Verformung der Betätigungseinheit auf.

Diese Art der Kraftmessung ist robust und mit verhältnismäßig geringem technischen Aufwand zu realisieren. Es besteht auch die Möglichkeit, existierende Systeme mit einem solchen Biegeelement nachzurüsten und damit auch an existierenden Systemen die Erfindung zu realisieren.

Bei einer weiteren vorteilhaften Ausgestaltung weist die Betätigungseinheit einen Wegsensor auf, der dafür ausgebildet ist, die aktuelle Stellung der Betätigungseinheit zwischen der Anfangsstellung und der Endstellung zu ermitteln.

Auf diese Weise lässt sich die aktuelle Stellung der Betätigungseinheit verhältnismäßig einfach messen.

Bei einer weiteren vorteilhaften Ausgestaltung kann der Grenzwert vom Benutzer eingestellt werden.

Dies bietet dem Benutzer insbesondere die Möglichkeit eine Anpassung bzgl. der Beschaffenheit des Objekts vorzunehmen, mit dem der Aktuator zusammenwirken soll. Dabei kann der Benutzer bei einem druckempfindlichen Objekt einen geringeren Grenzwert wählen als bei einem weniger druckempfindlichen oder härterem Objekt.

Bei einer weiteren vorteilhaften Ausgestaltung ist der Grenzwert durch eine Begrenzung des Stroms realisiert, der an den Aktuatormotor geleitet wird.

Auf diese Weise lässt sich der Grenzwert verhältnismäßig einfach realisieren. Dies gilt insbesondere dann, sofern die konkrete praktische Anwendung eine direkte Messung am Aktuator nicht oder nur aufwendig zulässt.

Bei einer weiteren vorteilhaften Ausgestaltung ist der Grenzwert über ein Schleifkupplung oder eine einstellbare Überlastfeder realisiert.

Auf diese Weise lässt sich der Grenzwert zuverlässig unter Rückgriff auf mechanische Mittel realisieren. Eine solche Realisierung kann dabei zusätzlich zu anderen Maßnahmen erfolgen, wie beispielsweise einer Begrenzung des Stroms, der an den Aktuatormotor geleitet wird, um eine Redundanz und Sicherheit zu erzielen.

Bei einer weiteren vorteilhaften Ausgestaltung weist die Betätigungseinheit einen ersten starren Griffschenkel und einen zweiten beweglichen Griffschenkel auf.

Die Aufgabe wird ferner gelöst durch ein nicht-chirurgisches Verfahren zum Ansteuern eines Aktuators eines endoskopischen Systems, insbesondere eines hier beschriebenen endoskopischen Systems, wobei ein Aktuatormotor, der mit dem Aktuator wirkverbunden ist: a) während einer Betätigung einer Betätigungseinheit des endoskopischen Systems zwischen einer Anfangsstellung und einer Endstellung in Abhängigkeit von einer aktuellen Stellung der Betätigungseinheit betätigt wird, wenn eine vom Aktuator ausgeübte Aktuatorkraft einen Grenzwert nicht überschreitet, b) während einer Betätigung der Betätigungseinheit zwischen der Anfangsstellung und der Endstellung nicht weiter betätigt wird, wenn eine vom Aktuator ausgeübte Aktuatorkraft den Grenzwert erreicht oder überschreitet, und c) während einer Betätigung der Betätigungseinheit in der Endstellung in Abhängigkeit von einer auf die Betätigungseinheit ausgeübten Bedienkraft über den Grenzwert hinaus angesteuert wird.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen. Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Ausführungsbeispiele der Erfindung sind in der Zeichnung dargestellt und werden in der nachfolgenden Beschreibung näher erläutert. Es zeigen:
- Fig. 1: eine Ausführungsform eines endoskopischen Systems, und
- Fig. 2: eine Ausführungsform eines Verfahrens zum Ansteuern eines Aktuators eines endoskopischen Systems.

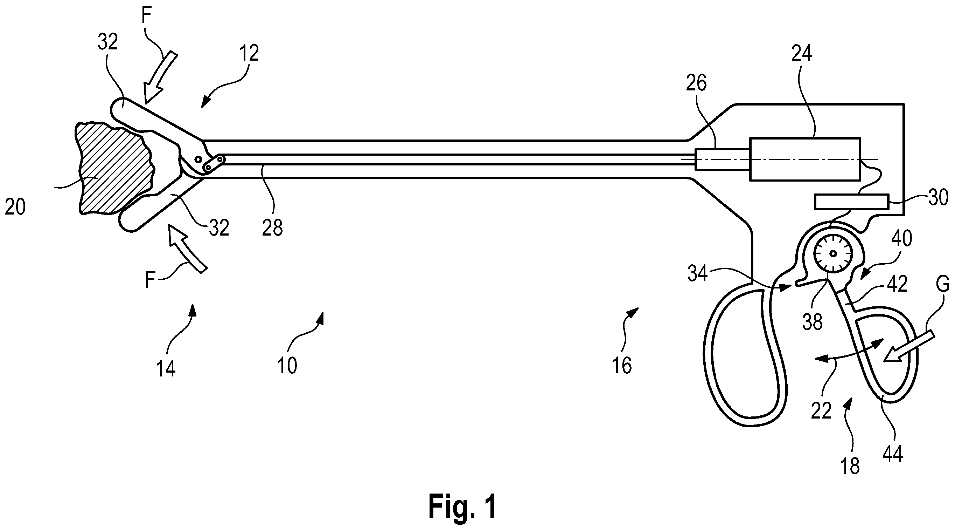

Fig. 1 zeigt eine Ausführungsform eines endoskopischen Systems 10 mit einen Aktuator 12 an einem distalen Ende 14 des Systems 10. Das endoskopische System 10 ist hier als endoskopisches Instrument ausgestaltet, das in seiner Gesamtheit vom Chirurgen handgeführt wird. Es sind aber auch andere Ausgestaltungen möglich, z.B. als Robotersystem, wobei dann das distale Ende 14 vom Chirurgen gesteuert wird aber nicht direkt vom Chirurgen handgeführt wird.

An einem proximalen Ende 16 des Systems 10 ist eine handbedienbare Betätigungseinheit 18 zum Betätigen des Aktuators 12 und Wählen einer Aktuatorkraft F angeordnet, mit der der Aktuator 12 auf ein Objekt 20 einwirken soll. Die Betätigungseinheit 18 ist zwischen einer Anfangsstellung und einer Endstellung verlagerbar, angedeutet durch einen Doppelpfeil 22, indem der Benutzer eine Bedienkraft G auf die Betätigungseinheit 18 ausübt. Aufgrund des Prinzips Kraft gleich Gegenkraft, ist die Bedienkraft G für den Benutzer auch zu spüren.

Das endoskopische System 10 weist ferner einen Aktuatormotor 24 auf, der dazu ausgebildet ist, den Aktuator 12 anzusteuern, hier über eine steuerbare Schleifkupplung 26 und eine Zug- / Schubstange 28. Ferner ist eine Steuereinheit 30 vorhanden, die dafür ausgebildet ist, den Aktuatormotor 24 anzusteuern.

In einem ersten Betriebszustand, bei dem die vom Aktuator 12 ausgeübte Aktuatorkraft F einen Grenzwert FTH nicht überschreitet, wird der Aktuator 12 während einer Betätigung der Betätigungseinheit 18 zwischen der Anfangsstellung und der Endstellung in Abhängigkeit von einer aktuellen Stellung der Betätigungseinheit 18 betätigt. Dies bedeutet, solange der Grenzwert FTH der Aktuatorkraft F, die der Aktuator 12 ausübt, nicht erreicht ist, führt eine fortlaufende Betätigung der Betätigungseinheit 18 zu einer fortlaufenden Betätigung des Aktuators 12.

Konkret wird die Betätigungseinheit 18 fortlaufend in Richtung der Endstellung betätigt, so schließen sich Maulteile 32 des Aktuators 12 immer weiter. Während dieses Betriebszustands verspürt der Benutzer nur eine geringe oder keine Gegenkraft durch die Betätigungseinheit 18.

In einem zweiten Betriebszustand hat die vom Aktuator 12 ausgeübte Aktuatorkraft F den Grenzwert FTH erreicht oder überschritten. Dies tritt in aller Regel dann ein, wenn der Aktuator 12 mit dem Objekt 20 in Kontakt getreten ist und begonnen hat auf das Objekt 20 einzuwirken. Dies führt dazu, dass eine fortlaufende Betätigung der Betätigungseinheit 18 nicht mehr zu einer fortlaufenden Betätigung des Aktuators 12 führt. Konkret, der Aktuator 12 bewegt sich nicht weiter.

Bei den hier beispielhaft gezeigten Maulteilen 32 bedeutet dies, dass die Maulteile 32 nicht weiter geschlossen werden, sondern in der aktuellen Position verbleiben. Dies führt üblicherweise auch dazu, dass die durch die Maulteile 32 ausgeübte Aktuatorkraft F unverändert bleibt. Mit anderen Worten, obwohl der Benutzer die Betätigungseinheit 18 weiterhin fortlaufend in Richtung der Endstellung betätigt, findet keine weitergehende Betätigung des Aktuators 12 statt.

In einem dritten Betriebszustand hat die Betätigungseinheit 18 die Endstellung erreicht. Die Endstellung kann insbesondere mittels eines Endanschlags 34 realisiert sein. Nun wird der Aktuator 12 in Abhängigkeit von einer auf die Betätigungseinheit 18 ausgeübten Aktuatorkraft F über den Grenzwert FTH hinaus angesteuert. Das heißt, in der Endstellung kann nun die durch den Aktuator 12 ausgeübte Aktuatorkraft F fein dosiert erhöht werden. Bei der hier gezeigten Ausführungsform wird die Schleifkupplung 26 weiter geschlossen, um die nun gewünschte größere Kraftübertragung zu ermöglichen.

Konkret können die Maulteile 32 nun noch weitergeschlossen werden, bis die gewünschte Greifsituation erreicht ist. Der Benutzer kann dabei den gewünschten Erfolg über einen Bildschirm, der den Arbeitsbereich des Aktuators 12 zeigt, prüfen. Gleichzeitig erhält der Benutzer aber auch eine direkte haptische Rückmeldung, weil er die Betätigungseinheit 18 mit der Bedienkraft G gegen den Endanschlag 34 drückt.

Bei dem hier gezeigten Ausführungsbeispiel wird die Verlagerung der Betätigungseinheit 18 im ersten und zweiten Betriebszustand mittels eines Wegsensors 38 erfasst und wird die Bedienkraft G an der Betätigungseinheit 18 im dritten Betriebszustand mittels eines Kraftsensors 40 erfasst, der ein Biegeelement 42 aufweist. Die Betätigungseinheit 18 weist einen Griffschenkel 44 auf.

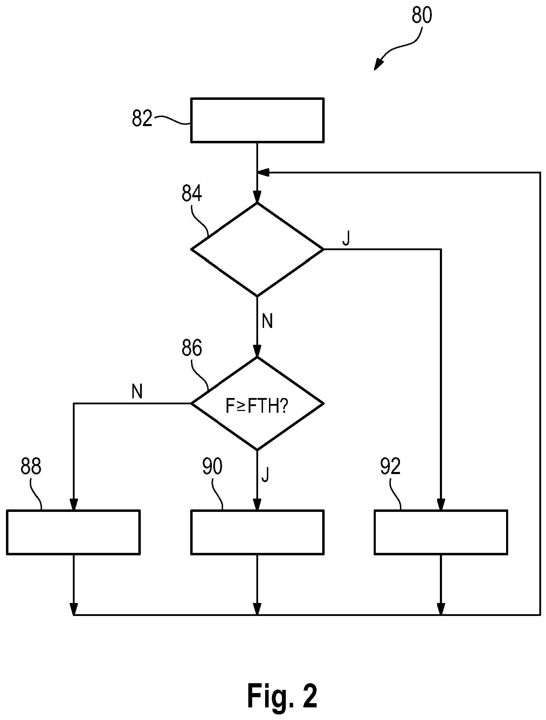

Fig. 2 zeigt eine Ausführungsform eines Verfahrens 80 zum Ansteuern eines Aktuators 12 eines endoskopischen Systems 10. Dabei sei angenommen, dass das Verfahren 80 mit dem Schritt 82 beginnt, in dem sich die Betätigungseinheit 18 in der Anfangsstellung befindet und danach aus der Anfangsstellung heraus in Richtung der Endstellung bewegt wird.

In einem Schritt 84 wird geprüft, ob die Endstellung erreicht ist. Ist dies nicht der Fall, verzweigt das Verfahren 80 über den N-Zweig zum Schritt 86. Hier wird geprüft, ob die aktuell vom Aktuator 12 ausgeübte Aktuatorkraft F den Grenzwert FTH erreicht oder überschritten hat. Ist dies nicht der Fall, verzweigt das Verfahren 80 über den N-Zweig zum Schritt 88. Dieser Schritt 88 entspricht dem ersten Betriebszustand, in dem eine fortlaufende Betätigung der Betätigungseinheit 18 zu einer fortlaufenden Betätigung des Aktuators 12 führt.

Ist die Prüfung im Schritt 86 positiv, verzweigt das Verfahren 80 über den J-Zweig zum Schritt 90. Dieser Schritt 90 entspricht dem zweiten Betriebszustand, in dem eine fortlaufende Betätigung der Betätigungseinheit 18 nicht mehr zu einer fortlaufenden Betätigung des Aktuators 12 führt.

Ist die Prüfung im Schritt 84 positiv, verzweigt das Verfahren 80 über den J-Zweig zum Schritt 92. Dieser Schritt 90 entspricht dem dritten Betriebszustand, in dem der Aktuator 12 in Abhängigkeit von einer auf die Betätigungseinheit 18 ausgeübten Bedienkraft G über den Grenzwert FTH hinaus angesteuert wird.

Bei einer Ausführungsform ist der technische Ablauf bevorzugt wie folgt. Zunächst wird die Betätigungseinheit 18 aus der Anfangsstellung in Richtung der Endstellung bewegt. Da der Griffschenkel 44 frei beweglich ist, ist an der Betätigungseinheit 18 keine oder nur eine minimale Bedienkraft G zu spüren.

Der Aktuatormotor 24 wird über den Wegsensor 38 und das Steuermodul 30 angesteuert, bewegt entsprechend die Übertragungsmechanik 26, 28 und schließt die Maulteile 32, so dass das Objekt 20, hier ein Gewebe, gegriffen wird.

Aufgrund der geringen Bedienkraft G bzw. Schließkraft an der Betätigungseinheit 18 wird über das Steuermodul 30 die Aktuatorkraft F bzw. Greifkraft der Maulteile 32 (z.B. über die Leistungsbegrenzung der Motorkraft des Aktuatormotors 24 und / oder die Einstellung der Schleifkupplung 26) stark begrenzt, so dass sich die Maulteile 32 nur so weit schließen, bis das Objekt 20 mit geringer Aktuatorkraft F bzw. Greifkraft gegriffen wird. Der Benutzer erkennt anhand der Deformation des Objekts, also insbesondere einer Gewebedeformation, wie hart oder weich das Objekt 20 bzw. Gewebe ist.

Da sich bei gegriffenem Objekt 20 wegen der geringen Aktuatorkraft F bzw. Greifkraft die Maulteile 32 nicht weiter schließen, der Griffschenkel 44 der Betätigungseinheit 18 aber frei beweglich sind, kann der Benutzer den Griffschenkel 44 vollends bis zum Endanschlag 34 schließen. Solange ändert sich nichts an der Aktuatorkraft F bzw. Greifkraft, da bis dahin keine oder nur eine minimale Bedienkraft G bzw. Schließkraft auf den Kraftsensor 40 wirkt.

Sobald die Betätigungseinheit 18 geschlossen ist bzw. die Endstellung erreicht hat, kann der Benutzer über den Griffschenkel 44 gegen den Endanschlag 34 eine erhöhte Bedienkraft G aufbringen. Diese wird über den Kraftsensor 40 und das Steuermodul 30 erfasst, über welche die Motorkraft des Aktuatormotors 24 und damit die Aktuatorkraft F bzw. Greifkraft auf das Objekt 20 entsprechend der vom Benutzer manuell aufgebrachten Bedienkraft G an der Betätigungseinheit 18 dann erhöht bzw. nachgeregelt wird.

Bei bevorzugten Ausführungsformen werden für die Kraftsensorik an der Betätigungseinheit 18 bzw. Eingabeeinheit unterschiedliche Varianten und Ausführungen eingesetzt. Dazu zählen neben unterschiedlichen Kraftsensoren 40 auch ein federndes Nachgeben bei Kraftaufbringung, die die Handhabung erleichtern könnte, da dann eine gewissenmaßen taktile Wegerfassung die Kraftdosierung bei der Eingabe unterstützt, und ein Federelement im Griffschenkel 44, so dass die Wegsensorik in Kombination mit einer Federkennlinie auch gleichzeitig zur Kraftmessung genutzt werden kann.

Bei bevorzugten Ausführungsformen werden andere Techniken zur Veränderung der Aktuatorkraft F bzw. Greifkraft eingesetzt. Anstatt über eine Motorsteuerung, insbesondere mittels einer Lastbegrenzung, die Aktuatorkraft F bzw. eine Greifkraft zu verändern, kann eine Veränderung der Vorspannung einer Überlastfeder über einen zweiten Motor gesteuert werden oder können eine Rutschkupplung, ein Getriebe und / oder ein Bremsmechanismus die gewünschte Rückmeldung bereitgestellt werden.

Bei bevorzugten Ausführungsformen wird eine zusätzliche, insbesondere distale, Wegmessung zur Optimierung der Steuerung eingesetzt. So kann die Betätigungseinheit 18 bzw. Eingabeeinheit blockiert werden, sobald sich der Aktuator 12, insbesondere die Maulteile 32, nicht mehr bewegen. Dies kann bevorzugt über einen Rastmechanismus, über eine Motorkraft bzw. Motorgegenkraft und / oder einen Bremsmechanismus erzielt werden.

Zudem kann es bevorzugt sein, ein Federelement in den Antriebsstrang zu integrieren. Dies ermöglich eine Messung des Federwegs, und die Aktuatorkraft F bzw. Greifkraft kann dann über die Motorbewegung in Kombination mit einer Federkennlinie eingestellt werden. Bevorzugt können auch verschiedene Messverfahren eingesetzt werden, insbesondere eine optische Wegmessung über ein bildgebendes System, dessen Bilder hinsichtlich einer Systemerkennung und / oder Bewegungserfassung ausgewertet werden, bevorzugt mit Hilfe von künstlicher Intelligenz.

In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Endoskopisches System 10 aufweisend einen Aktuator 12 an einem distalen Ende 14 des Systems 10, eine handbedienbare Betätigungseinheit 18 an einem proximalen Ende 16 des Systems 10 zum Betätigen des Aktuators 12 und Wählen einer Aktuatorkraft F, mit der der Aktuator 12 auf ein Objekt 20 einwirken soll, wobei die Betätigungseinheit 18 zwischen einer Anfangsstellung und einer Endstellung verlagerbar ist, einen Aktuatormotor 24, der dazu ausgebildet ist, den Aktuator 12 anzusteuern, und eine Steuereinheit 30, die dafür ausgebildet ist, den Aktuatormotor 24 zunächst auf Basis eines Wegsensors 38 ohne Überschreitung eines Grenzwerts FTH und dann auf Basis eines Kraftsensors 40 anzusteuern. Ferner wird ein Verfahren zum Ansteuern eines Aktuators 12 eines endoskopischen Systems 10 offenbart.

## Patentansprüche

1. Endoskopisches System (10) aufweisend
- einen Aktuator (12) an einem distalen Ende (14) des Systems (10),
- eine handbedienbare Betätigungseinheit (18) an einem proximalen Ende (16) des Systems (10) zum Betätigen des Aktuators (12) und Wählen einer Aktuatorkraft (F), mit der der Aktuator (12) auf ein Objekt (20) einwirken soll, wobei die Betätigungseinheit (18) zwischen einer Anfangsstellung und einer Endstellung verlagerbar ist,
- einen Aktuatormotor (24), der dazu ausgebildet ist, den Aktuator (12) zum Erreichen einer gewünschten Stellung anzusteuern, und
- eine Steuereinheit (30), **dadurch gekennzeichnet dass**, die Steuereinheit (30) dafür ausgebildet ist, den Aktuatormotor (24)
a) während einer Betätigung der Betätigungseinheit (18) zwischen der Anfangsstellung und der Endstellung in Abhängigkeit von einer aktuellen Stellung der Betätigungseinheit (18) zu betätigen, wenn eine vom Aktuator (12) ausgeübte Aktuatorkraft (F) einen Grenzwert (FTH) nicht überschreitet,
b) während einer Betätigung der Betätigungseinheit (18) zwischen der Anfangsstellung und der Endstellung nicht weiter zu betätigen, wenn eine vom Aktuator (12) ausgeübte Aktuatorkraft (F) den Grenzwert (FTH) erreicht oder überschreitet, und
c) während einer Betätigung der Betätigungseinheit (18) in der Endstellung in Abhängigkeit von einer auf die Betätigungseinheit (18) ausgeübten Bedienkraft (G) über den Grenzwert (FTH) hinaus anzusteuern.

2. Endoskopisches System (10) nach Anspruch 1, wobei das System für eine Zangenfunktion ausgebildet ist, der Aktuator (12) zwei Maulteile (32) aufweist und das Betätigen des Aktuators (12) ein Öffnen und Schließen der Maulteile (32) ist.

3. Endoskopisches System (10) nach einem der vorhergehenden Ansprüche, wobei die Betätigungseinheit (18) einen Kraftsensor (40) aufweist, der dafür ausgebildet ist, zumindest in der Endstellung die auf die Betätigungseinheit (18) ausgeübte Bedienkraft (G) zu messen.

4. Endoskopisches System (10) nach Anspruch 3, wobei der Kraftsensor ein Biegeelement zur Messung einer Verformung der Betätigungseinheit (18) aufweist.

5. Endoskopisches System (10) nach einem der vorhergehenden Ansprüche, wobei die Betätigungseinheit (18) einen Wegsensor (38) aufweist, der dafür ausgebildet ist, die aktuelle Stellung der Betätigungseinheit (18) zwischen der Anfangsstellung und der Endstellung zu ermitteln.

6. Endoskopisches System (10) nach einem der vorhergehenden Ansprüche, wobei der Grenzwert (FTH) vom Benutzer eingestellt werden kann.

7. Endoskopisches System (10) nach einem der vorhergehenden Ansprüche, wobei der Grenzwert (FTH) durch eine Begrenzung des Stroms realisiert ist, der an den Aktuatormotor (24) geleitet wird.

8. Endoskopisches System (10) nach einem der vorhergehenden Ansprüche, wobei der Grenzwert (FTH) über ein Schleifkupplung (26) oder eine einstellbare Überlastfeder realisiert ist.

9. Endoskopisches System (10) nach einem der vorhergehenden Ansprüche, wobei die Betätigungseinheit (18) einen beweglichen Griffschenkel (44) aufweist.

10. Nicht-chirurgisches Verfahren zum Ansteuern eines Aktuators (12) eines endoskopischen Systems (10), wobei ein Aktuatormotor (24), der mit dem Aktuator (12) wirkverbunden ist,
a) während einer Betätigung einer Betätigungseinheit (18) des endoskopischen Systems zwischen einer Anfangsstellung und einer Endstellung in Abhängigkeit von einer aktuellen Stellung der Betätigungseinheit (18) betätigt wird, wenn eine vom Aktuator (12) ausgeübte Aktuatorkraft einen Grenzwert nicht überschreitet,
b) während einer Betätigung der Betätigungseinheit (18) zwischen der Anfangsstellung und der Endstellung nicht weiter betätigt wird, wenn eine vom Aktuator (12) ausgeübte Aktuatorkraft den Grenzwert erreicht oder überschreitet, und
c) während einer Betätigung der Betätigungseinheit (18) in der Endstellung in Abhängigkeit von einer auf die Betätigungseinheit (18) ausgeübten Bedienkraft (G) über den Grenzwert (FTH) hinaus angesteuert wird.

## Claims

1. An endoscopic system (10) having
- an actuator (12) at a distal end (14) of the system (10),
- a manually operable actuating unit (18) at a proximal end (16) of the system (10) for actuating the actuator (12) and selecting an actuator force (F) with which the actuator (12) is to act on an object (20), wherein the actuating unit (18) is displaceable between an initial position and an end position,
- an actuator motor (24) designed to activate the actuator (12) to reach a desired position, and
- a control unit (30) **characterised in that** the control unit (30) is designed to actuate the actuator motor (24)
a) during an actuation of the actuating unit (18) between the initial position and the end position as a function of a current position of the actuating unit (18), if an actuator force (F) exerted by the actuator (12) does not exceed a limit value (FTH),
b) to not actuate the motor any further during an actuation of the actuating unit (18) between the initial position and the end position, if an actuator force (F) exerted by the actuator (12) reaches or exceeds the limit value (FTH), and
c) to activate the motor beyond the limit value (FTH) during an actuation of the actuating unit (18) in the end position as a function of an operating force (G) exerted on the actuating unit (18).

2. The endoscopic system (10) according to claim 1, wherein the system is designed for a forceps function, the actuator (12) has two jaw parts (32), and the actuation of the actuator (12) is an opening and closing of the jaw parts (32).

3. The endoscopic system (10) according to one of the preceding claims, wherein the actuating unit (18) has a force sensor (40) which is designed to measure the operating force (G) exerted on the actuating unit (18) at least in the end position.

4. The endoscopic system (10) according to claim 3, wherein the force sensor has a bending element for measuring a deformation of the actuating unit (18).

5. The endoscopic system (10) according to one of the preceding claims, wherein the actuating unit (18) has a displacement sensor (38) which is designed to determine the current position of the actuating unit (18) between the initial position and the end position.

6. The endoscopic system (10) according to one of the preceding claims, wherein the limit value (FTH) can be set by the user.

7. The endoscopic system (10) according to one of the preceding claims, wherein the limit value (FTH) is realised by limiting the current conducted to the actuator motor (24).

8. The endoscopic system (10) according to one of the preceding claims, wherein the limit value (FTH) is realised via a slip coupling (26) or an adjustable overload spring.

9. The endoscopic system (10) according to one of the preceding claims, wherein the actuating unit (18) has a movable grip member (44).

10. A non-surgical method for activating an actuator (12) of an endoscopic system (10), wherein an actuator motor (24), which is operatively connected to the actuator (12),
a) is actuated during an actuation of an actuating unit (18) of the endoscopic system between an initial position and an end position as a function of a current position of the actuating unit (18), if an actuator force exerted by the actuator (12) does not exceed a limit value,
b) is not actuated any further during an actuation of the actuating unit (18) between the initial position and the end position, if an actuator force exerted by the actuator (12) reaches or exceeds the limit value, and
c) is activated beyond the limit value (FTH) during an actuation of the actuating unit (18) in the end position as a function of an operating force (G) exerted on the actuating unit (18).

## Revendications

1. Système endoscopique (10) présentant
- un actionneur (12) au niveau d'une extrémité distale (14) du système (10),
- une unité d'actionnement (18) à activation manuelle au niveau d'une extrémité proximale (16) du système (10) pour actionner l'actionneur (12) et sélectionner une force d'actionneur (F), avec laquelle l'actionneur (12) doit agir sur un objet (20), dans lequel l'unité d'actionnement (18) peut être déplacée entre une position initiale et une position finale,
- un moteur d'actionneur (24), qui est conçu, pour commander l'actionneur (12) pour atteindre une position souhaitée, et
- une unité de commande (30) **caractérisé en ce que**, l'unité de commande (30) est conçue, pour actionner le moteur de l'actionneur (24)
a) pendant un actionnement de l'unité d'actionnement (18), entre la position initiale et la position finale, en fonction d'une position actuelle de l'unité d'actionnement (18), lorsqu'une force d'actionneur (F) exercée par l'actionneur (12) ne dépasse pas une valeur limite (FTH),
b) pour ne pas actionner le moteur davantage pendant un actionnement de l'unité d'actionnement (18) entre la position initiale et la position finale, lorsqu'une force d'actionneur (F) exercée par l'actionneur (12) atteint ou dépasse la valeur limite (FTH), et
c) pour commander le moteur pendant un actionnement de l'unité d'actionnement (18) dans la position finale, en fonction d'une force d'activation (G) exercée sur l'unité d'actionnement (18), au-delà de la valeur limite (FTH).

2. Système endoscopique (10) selon la revendication 1, dans lequel le système est conçu pour une fonction de pince, l'actionneur (12) présente deux parties de mâchoire (32) et l'actionnement de l'actionneur (12) est une ouverture et une fermeture des parties de mâchoire (32).

3. Système endoscopique (10) selon l'une des revendications précédentes, dans lequel l'unité d'actionnement (18) présente un capteur de force (40), qui est conçu, pour mesurer, au moins dans la position finale, la force d'activation (G) exercée sur l'unité d'actionnement (18).

4. Système endoscopique (10) selon la revendication 3, dans lequel le capteur de force présente un élément de flexion pour mesurer une déformation de l'unité d'actionnement (18).

5. Système endoscopique (10) selon l'une des revendications précédentes, dans lequel l'unité d'actionnement (18) présente un capteur de déplacement (38), qui est conçu, pour déterminer la position actuelle de l'unité d'actionnement (18) entre la position initiale et la position finale.

6. Système endoscopique (10) selon l'une des revendications précédentes, dans lequel la valeur limite (FTH) peut être réglée par l'utilisateur.

7. Système endoscopique (10) selon l'une des revendications précédentes, dans lequel la valeur limite (FTH) est réalisée par une limitation du courant, qui est dirigé vers le moteur d'actionneur (24).

8. Système endoscopique (10) selon l'une des revendications précédentes, dans lequel la valeur limite (FTH) est réalisée par l'intermédiaire d'un accouplement à frottement (26) ou d'un ressort de surcharge réglable.

9. Système endoscopique (10) selon l'une des revendications précédentes, dans lequel l'unité d'actionnement (18) présente une branche de poignée mobile (44).

10. Procédé non chirurgical de commande d'un actionneur (12) d'un système endoscopique (10), dans lequel un moteur d'actionneur (24), qui est en liaison fonctionnelle avec l'actionneur (12),
a) est actionné pendant un actionnement d'une unité d'actionnement (18) du système endoscopique entre une position initiale et une position finale en fonction d'une position actuelle de l'unité d'actionnement (18), lorsqu'une force d'actionneur exercée par l'actionneur (12) ne dépasse pas une valeur limite,
b) n'est pas actionné davantage pendant un actionnement de l'unité d'actionnement (18) entre la position initiale et la position finale, lorsqu'une force d'actionneur exercée par l'actionneur (12) atteint ou dépasse la valeur limite, et
c) est commandé pendant un actionnement de l'unité d'actionnement (18) dans la position finale en fonction d'une force d'activation (G) exercée sur l'unité d'actionnement (18) au-delà de la valeur limite (FTH).
